(19) Europäisches Patentamt
European Patent Office
Office européen des brevets

(11) **EP 2 626 107 B1**

(12) **EUROPEAN PATENT SPECIFICATION**

(45) Date of publication and mention
of the grant of the patent:
**26.05.2021 Bulletin 2021/21**

(21) Application number: **11830748.7**

(22) Date of filing: **06.10.2011**

(51) Int Cl.:
***A61M 37/00*** *(2006.01)*

(86) International application number:
**PCT/JP2011/073129**

(87) International publication number:
**WO 2012/046816 (12.04.2012 Gazette 2012/15)**

(54) **APPLICATOR**

APPLIKATOR

APPLICATEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.10.2010 JP 2010227684**

(43) Date of publication of application:
**14.08.2013 Bulletin 2013/33**

(73) Proprietor: **Hisamitsu Pharmaceutical Co., Inc.
Tosu-shi, Saga 841-0017 (JP)**

(72) Inventors:
• **TOKUMOTO, Seiji
Tsukuba-shi
Ibaraki 305-0856 (JP)**
• **OGURA, Makoto
Tsukuba-shi
Ibaraki 305-0856 (JP)**
• **MACHIDA, Kazuya
Tsukuba-shi
Ibaraki 305-0856 (JP)**

(74) Representative: **Grünecker Patent- und
Rechtsanwälte
PartG mbB
Leopoldstraße 4
80802 München (DE)**

(56) References cited:
**JP-A- 2005 533 625    JP-A- 2007 516 781
US-A1- 2005 096 586    US-A1- 2005 261 631**

## Description

## Technical Field

One aspect of the present invention relates to an applicator which is used to aid administering an active ingredient with microneedle.

## Background Art

There has been known an applicator which applies, to a skin, a microneedle for administering an active ingredient via a skin by impacting the microneedle.

For example, JP 4198985 B2 discloses an applicator including an apparatus main body, a piston for causing a penetrating member to collide with a stratum corneum, an impact spring for providing an impact force to the piston, a presser bar spring acting between the apparatus main body and a cap, and a locking mechanism for cocking the piston by compressing the apparatus main body and piston together using a compressive force and locking the piston in a cocked position.

JP 2007-509706 A discloses an applicator having a housing, a piston movable within the housing, and a cap.

JP 2010-501211 A discloses an puncturing device for puncture with a puncturing head including an infusion cannula or a puncturing tip to puncture a body of a patient. The device includes two actuation members which need to be actuated simultaneously to trigger puncturing movement.

JP 2010-233803 A discloses a puncturing apparatus in which at least one end of a drive spring is arranged so as to be fixed to neither a housing-side abutment portion nor a piston.

US 2005/0261631 A1 and JP 2005-533625 A disclose a plunger-type microneedle delivery apparatus according to the preamble of claim 1.

## Summary of Invention

## Technical Problem

However, the conventional applicators as disclosed in the above patent literatures are not only large in size and less portable but also may be detrimental to use in children and infants. There is thus a need to reduce the size and weight of an applicator.

Under the circumstances, one aspect of the present invention has an object to reduce the size and weight of an applicator.

## Solution to Problem

The invention is defined by the applicator of claim 1. Embodiments and optional features are defined in the dependent claims. An applicator according to the present invention is an applicator for applying a microneedle to a skin, including a tubular housing, a piston which transmits a biasing force of a biasing member to the microneedle, fixation means for fixing the piston against the biasing force of the biasing member in the housing, and a cap provided near a first end of the housing, the cap having a release mechanism for releasing the piston fixed by the fixation means at a surface facing the piston, wherein the cap is provided to be freely movable in an extending direction of the housing such that the release mechanism can contact the fixation means when the piston is fixed by the fixation means.

According to the invention, the cap for releasing the piston fixed against the biasing force of the biasing member is provided to be freely movable in the extending direction of the housing. Thus, a member for fixing the cap to the housing, a presser bar spring acting between an apparatus main body and the cap, and the like can be omitted, and the applicator can be reduced in size and weight accordingly.

In the applicator according to another form, a whole of the cap may be housed in the housing. In this case, the configuration in which the cap is housed in the housing reduces a dimension in a width direction of the applicator. This allows reduction in the size and weight of the applicator.

In the applicator according to still another form, an opening at a second end of the housing may have a same shape as a shape of the microneedle and have dimensions not smaller than dimensions of the microneedle. In this case, the applicator can be reduced in size according to a shape and dimensions of the microneedle. Additionally, since the applicator is unlikely to be displaced in the width direction relative to the microneedle, the piston can be put on the microneedle while the piston keeps a parallel positional relationship with the microneedle. This allows reliable puncture (enhances the reproducibility of puncture).

In the applicator according to still another form, the biasing member may be a conical spring. This configuration allows reduction in length in an extending/contracting direction of the spring at compression. It is thus possible to reduce a dimension in the extending direction of the housing and reduce size and weight of the applicator.

In the applicator according to still another form, a partition wall for separating a first space housing the release mechanism of the cap and a second space housing the piston from each other may be provided in the housing, and the fixation means may be composed of a rim of a through hole which is formed in the partition wall and a claw portion which is provided at an end of a piston rod of the piston and can engage with the rim. By arranging the fixation means in such a simple manner, it is possible to reduce the number of components required for the applicator and reduce size and weight of the applicator accordingly.

In the applicator according to still another form, the claw portion may be formed to taper down toward the end of the piston rod. The thus-formed claw portion can be engaged with the rim of the through hole with a small force, and the piston can be easily fixed.

In the applicator according to still another form, the release mechanism may be a projecting portion which is provided at the surface of the cap facing the piston, and a recessed portion for disengaging the claw portion from the rim of the partition wall by pushing the claw portion toward a center of the through hole may be formed in the projecting portion. The release mechanism arranged in this manner allows reduction in the number of components required for the applicator. Additionally, overlap between the claw portion and the release mechanism allows reduction in size and weight of the applicator.

In the applicator according to still another form, the recessed portion formed in the projecting portion may have a mortar-shape. With this configuration, the claw portion is drawn along the mortar-shape, and the fixed piston is released while the piston rod keeps parallel to an inner wall of the housing. This allows reliable puncture (enhances the reproducibility of puncture).

In the applicator according to the invention, the biasing member may be an elastic member, and the piston may move without being subjected to the biasing force of the elastic member in a first zone which is a part of a movement zone for the piston moving toward the skin.

The applicator according to still another form may include a support base which supports the elastic member and transmits the biasing force to the piston in a second zone different from the first zone, and the support base that pushes the piston by the biasing force may stop at one end of the second zone to allow the piston to move without being subjected to the biasing force in the first zone.

**Advantageous Effects of Invention**

According to one aspect of the present invention, a member for fixing a cap for releasing a piston fixed against a biasing force to a housing, a presser bar spring acting between an apparatus main body and the cap, and the like are unnecessary. An applicator can be reduced in size and weight by an amount corresponding to the components.

**Brief Description of Drawings**

[Figure 1] Figure 1 is a perspective view of an applicator according to a first embodiment as seen from above.
[Figure 2] Figure 2 is a perspective view of the applicator shown in Figure 1 as seen from below.
[Figure 3] Figure 3 is a perspective view of a piston shown in Figure 2.

[Figure 4] Figure 4 is a perspective view of a cap shown in Figure 1.
[Figure 5] Figures 5(a) and 5(b) are cross-sectional views taken along line V-V in Figure 2 when a spring shaped like a circular column is used, Figure 5(a) shows a state in which the piston is biased, and Figure 5(b) shows a state after the piston is actuated.
[Figure 6] Figures 6(a) and 6(b) are cross-sectional views taken along line V-V in Figure 2 when a conical spring is used, and Figures 6(a) and 6(b) show a state in which the piston is biased and a state after the piston is actuated, respectively.
[Figure 7] Figure 7 is a view for explaining a case where an aid is used during use of the applicator shown in Figure 1.
[Figure 8] Figure 8 is a view for explaining how to use the applicator shown in Figure 1.
[Figure 9] Figure 9 is a perspective view of an applicator according to a second embodiment as seen from above.
[Figure 10] Figure 10 is a perspective view of the applicator shown in Figure 9 as seen from below.
[Figure 11] Figure 11 is a perspective view of a piston shown in Figure 10.
[Figure 12] Figure 12 is a cross-sectional view taken along line XII-XII in Figure 10.
[Figure 13] Figure 13 is a cross-sectional view representing a state in which claw portions shown in Figure 12 are fixed.
[Figure 14] Figure 14 is a view for explaining how to use the applicator in Figure 9.
[Figure 15] Figure 15 is a graph showing the amounts of OVA transferred when two types of applicators (type A and type B) according to an example are used.
[Figure 16] Figure 16 is a graph showing a velocity profile of a piston in type B according to the example.
[Figure 17] Figure 17 is a perspective view of an applicator according to a modification.
[Figure 18] Figure 18(a) is a perspective view of an applicator according to another modification, and Figure 18(b) is a cross-sectional view taken along line B-B in Figure 18(a).
[Figure 19] Figure 19 is a perspective view of an applicator according to still another modification from above.
[Figure 20] Figure 20 is a cross-sectional view taken along line XX-XX in Figure 19.
[Figure 21] Figure 21 is a cross-sectional view representing a state in which claw portions shown in Figure 20 are fixed.

**Description of Embodiments**

Embodiments of the present invention will be described in detail below with reference to the accompanying drawings. Note that identical or equivalent elements are denoted by same reference numerals in the drawings, and redundant description thereof will be omitted.

(First Embodiment)

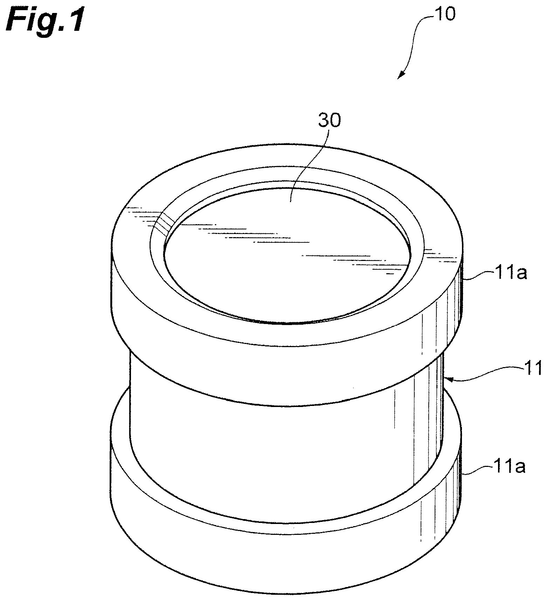

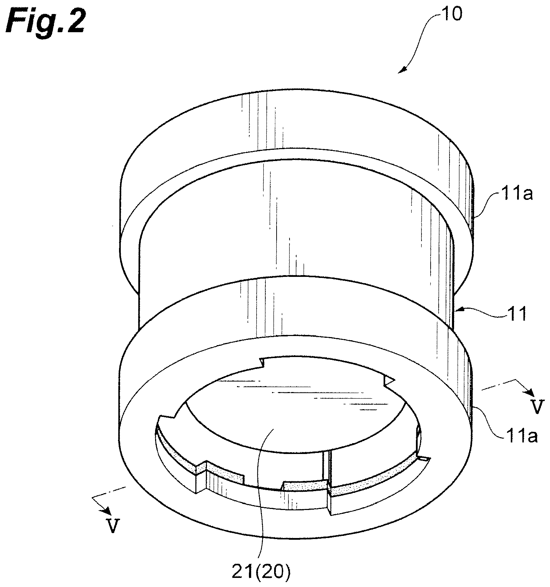

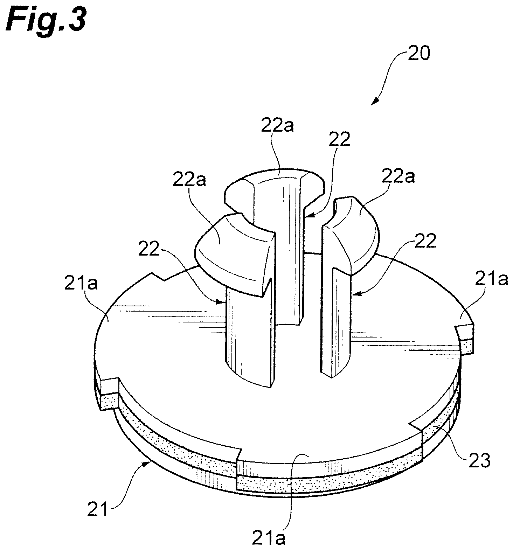

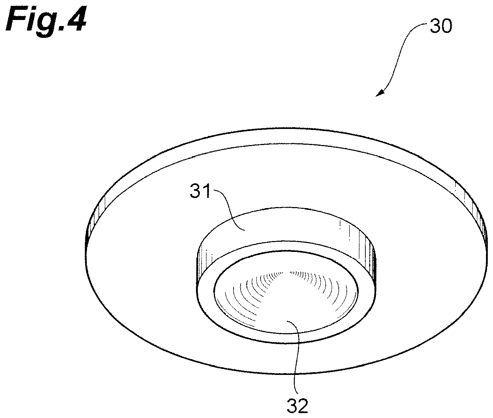

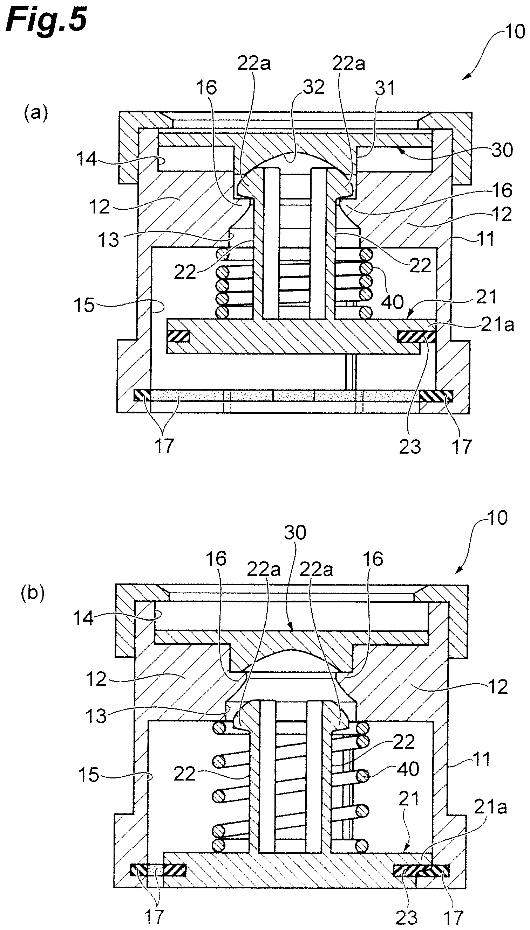

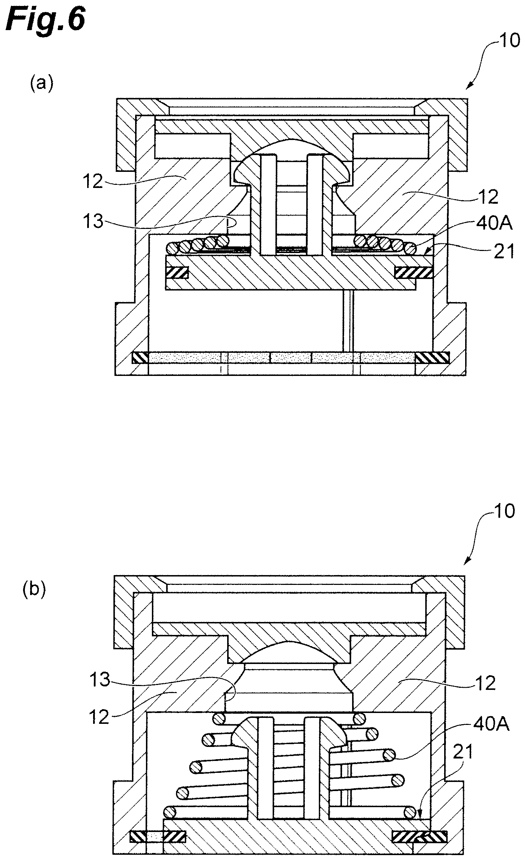

The structure of an applicator 10 according to a first embodiment will first be described with reference to Figures 1 to 6. Figure 1 is a perspective view of the applicator 10 from above. Figure 2 is a perspective view of the applicator 10 from below. Figure 3 is a perspective view of a piston 20. Figure 4 is a perspective view of a cap 30. Figures 5 and 6 are cross-sectional views taken along line V-V in Figure 2.

A housing 11 of the applicator 10 has a circular tube shape. Annular members 11a which fit on the housing 11 at two ends can be said to be a part of the housing 11. Note that the applicator 10 desirably has a shape easy to grip and apply microneedles to a skin. For example, a tubular housing having a polygonal cross section may be used, an outer wall may have a generally rounded shape, or the tubular housing may be dented or stepped. Alternatively, fine grooves may be formed in or a coating may be applied to a surface of the housing in order to resist slippage.

The piston 20 for impacting microneedles put on a skin is housed near one end (a second end) in the housing 11 (see Figure 2). The cap 30 for actuating the piston 20 is housed near the other end (a first end) in the housing 11 (see Figure 1). In the following description, the side where the cap 30 is housed is defined as the upper side of the applicator 10 or the housing 11, and the side where the piston 20 is housed is defined as the lower side of the applicator 10 or the housing 11.

As shown in Figure 3, the piston 20 is composed of a disc-like piston plate 21 which is to collide with microneedles and a plurality of piston rods 22 which extend from near the center of an upper surface of the piston plate 21 in a direction orthogonal to the upper surface. The piston plate 21 projects outward in a radial direction at several points of a rim (projecting portions 21a). The piston plate 21 has substantially the same diameter as the inner diameter of the housing 11. The piston plate 21 has a notch formed at a central portion in an axial direction, and a cushioning material 23 is provided in the notch and on lower surfaces of the projecting portions 21a. Examples of the material for the cushioning material 23 include an elastic body such as rubber or silicon, and silicon is preferable in view of age-related deterioration. The material for the cushioning material 23, however, is not particularly limited. A hooked claw portion 22a projecting outward in the radial direction of the piston plate 21 is formed at a distal end of each piston rod 22. The claw portion 22a tapers down toward the distal end of the piston rod 22.

As shown in Figure 4, the cap 30 is disc-like and has substantially the same diameter as the inner diameter of the housing 11. A projecting portion 31 in the shape of a circular column is provided at a central portion of a lower surface of the cap 30 and has a recessed portion 32. The recessed portion 32 has a mortar-shape so as to receive the claw portions 22a of the piston rods 22.

As shown in Figures 5, a partition wall 12 for separating the inside into an upper portion and a lower portion is provided at a portion near the top of the inside of the housing 11. In the following description, a space on the upper side of the partition wall 12 is referred to as an upper space (a first space) 14 while a space on the lower side is referred to as a lower space (a second space) 15. A circular through hole 13 is formed in a central portion of the partition wall 12, and the upper space 14 and lower space 15 are connected via the through hole 13. A rim of the through hole 13 projects inward more near a central portion than at an upper portion and at a lower portion in cross section.

An upper portion of the through hole 13 is responsible for guiding the projecting portion 31 of the cap 30 and has substantially the same diameter as the diameter of the projecting portion 31. A projecting portion near a central portion of the through hole 13 is responsible for engaging with the claw portions 22a of the piston rods 22 to fix the piston 20 and is hereinafter referred to as a claw receiving portion 16. The claw receiving portion 16 is tapered on the lower side to spread downward. A lower portion of the through hole 13 is responsible for guiding the claw portions 22a of the piston rods 22.

The piston 20 is housed in the lower space 15. When the piston 20 is to be housed, one end of a compression spring (a biasing member or an elastic member, hereinafter to be simply referred to as a "spring") 40 which accumulates energy when compressed is attached to the lower space 15 side of the partition wall 12. The spring 40 has a diameter larger than the diameter of the lower portion of the through hole 13 and is thus attached so as to surround the through hole 13. The piston 20 is housed in the lower space 15 such that all the piston rods 22 fit inside the spring 40. The other end of the spring 40 is in contact with the upper surface of the piston plate 21.

After the piston 20 is housed in the lower space 15, the annular member 11a is attached to the lower end of the housing 11. A part of the annular member 11a is shaped so as to contact the projecting portions 21a of the piston plate 21 moving to outside the housing 11 by a biasing force of the spring 40. The configuration prevents the piston 20 from popping out of the housing 11. A cushioning material 17 is provided at a portion of the housing 11 (the annular member 11a) which contacts the projecting portions 21a of the piston plate 21. Examples of the material for the cushioning material 17 include an elastic body such as rubber or silicon, and silicon is preferable in view of age-related deterioration. The material for the cushioning material 17, however, is not particularly limited. The cushioning material 23 on the piston 20 side and the cushioning material 17 on the housing 11 side can reduce collision noise generated when the actuated piston 20 stops at the lower end of the housing 11.

The piston 20 can be fixed against the biasing force of the spring 40 in the lower space 15. If the piston plate 21 is pushed from outside the housing 11 against the biasing force of the spring 40 when the applicator 10 is in the state in Figure 5(b), the claw portions 22a of the piston rods 22 come into contact with the claw receiving portion 16. When the piston plate 21 is further pushed, the claw portion 22a and its surroundings of each piston rod 22 are bent toward the center of the through hole 13, the claw portion 22a reaches the upper portion of the through hole 13 beyond the claw receiving portion 16. As shown in Figure 5(a), the claw portions 22a are caught on the claw receiving portion 16, which causes the piston 20 to be fixed against the biasing force of the spring 40. Accordingly, the claw receiving portion 16 (the rim of the through hole 13) and the claw portions 22a of the piston rods 22 can be said to be fixation means. Note that the fixation of the piston 20 is also referred to as cocking.

The cap 30 is housed in the upper space 14 such that the projecting portion 31 faces the partition wall 12. After the whole of the cap 30 is housed in this manner, the annular member 11a is attached to an end of the cap 30. The configuration prevents the cap 30 from popping out of the housing 11.

Since the position of the cap 30 is not fixed in the upper space 14, the cap 30 can move freely in an extending direction of the housing 11 (a vertical direction) in the upper space 14. Accordingly, if the applicator 10 is tilted such that the cap 30 is located on the piston 20 that is fixed against the biasing force of the spring 40, the projecting portion 31 of the cap 30 comes into contact with the claw portions 22a of the piston 20, as shown in Figure 5(a). That is, the cap 30 is provided to be freely movable in the extending direction of the housing 11 in the upper space 14 such that the projecting portion 31 can contact the claw portions 22a when the piston 20 is fixed by the claw portions 22a. As described above, since a presser bar spring or the like can be omitted at the time of incorporation of the cap 30 into the housing 11, the number of components can be reduced.

Parameters related to energy of the piston 20 actuated by the biasing force of the spring 40 include a modulus of transverse elasticity, wire diameter, the number of turns, mean diameter of coil, distance indicating how much the spring 40 is compressed from its natural length, piston speed, spring mass, and piston mass.

The modulus of transverse elasticity of a spring depends on the material for the spring. The modulus of transverse elasticity is 68500 N/mm² when the material is stainless steel and is 78500 N/mm² when the material is piano wire (iron). Assumed values of the other parameters are as follows. The wire diameter is 0.1 to 5 mm, the number of turns is 1 to 20, the mean diameter of coil is 1 to 30 mm, the distance is 1 to 100 mm, the speed is 0.1 to 50 m/s, the spring mass is 0.1 to 5 g, and the piston mass is 0.1 to 20 g.

Spring- and piston-related theoretical formulae are defined using the above-described parameters as follows. Expression (1) indicates the relationship among a spring constant, the shape of a spring, and the material for the spring, and Expression (2) indicates the relationship between the mass and dimensions of the spring. Expression (3) indicates the relationship between the energy of the spring and kinetic energy, and Expression (4) indicates the relationship among the speed, energy, and mass of a piston. In the expressions below, G is a modulus (N/m²) of transverse elasticity, d is a wire diameter (m), n is the number of turns, D is a mean diameter (m) of coil, k is a spring constant (N/m), x is a distance (m), v is a speed (m/s), 1 is the length (m) of a spring when the spring is stretched, $\rho$ is a density (kg/m³), m is the mass (kg) of the spring, and M is the mass (kg) of a piston.

[Expression 1]

$$k = \frac{Gd^4}{8nD^3} \quad \ldots(1)$$

[Expression 2]

$$m = \frac{\rho \pi l d^2}{4} \quad \ldots(2)$$

[Expression 3]

$$E = \frac{1}{2}kx^2 = \frac{1}{2}Mv^2 \quad \ldots(3)$$

[Expression 4]

$$v = \sqrt{\frac{2E}{M}} \quad \ldots(4)$$

The spring 40 shown in Figures 5 is spirally wound in the shape of a circular tube. The shape of a spring is not limited to this. For example, a spring spirally wound in the shape of a cone (also referred to as a conical spring in this specification) 40A as shown in Figures 6 may be used. In this case, the spring 40A is attached to the partition wall 12 such that an end having a smaller diameter (an end corresponding to a portion near the point of a cone) of the spring 40A covers the through hole 13, and an end having a larger diameter (an end corresponding to a bottom surface of the cone) contacts the upper surface of the piston plate 21. When the conical spring 40A is used, the length in an extending/contracting direction of the spring at compression can be minimized up to the wire diameter of the spring. It is thus possible to reduce a dimension (the height) of the housing 11 with consideration of numerical values derived from Expressions (1) to (4) above and reduce the size and weight of the applicator 10.

The dimensions of the applicator 10 can be determined according to the dimensions of microneedles in the manner below. Note that a method for determining the dimensions is not limited to the one below.

The dimensions of the applicator 10 can be reduced according to the dimensions of microneedles by tailoring the shape of a lower opening of the housing 11 to the shape of the microneedles and tailoring the minimum inner diameter of the opening to the outer diameter of the microneedles. If the lower opening is shaped in the above-described manner, the applicator 10 is not displaced in a radial direction (width direction) relative to the microneedles when the applicator 10 is positioned on the microneedles, and the piston 20 can be put on the microneedles while the piston 20 keeps a parallel positional relationship with the microneedles. This allows reliable puncture (enhances the reproducibility of puncture). The minimum inner diameter of the opening may, of course, be not less than the outer diameter of the microneedles.

For example, the applicator 10 in which the minimum inner diameter of the lower end of the housing 11 is about 26 mm, and the outer diameter and height of the housing 11 are about 30 mm may be fabricated for microneedles having a diameter of 26 mm. The dimensions of the applicator 10 are, of course, not limited to the example. For example, the outer diameter may be determined within the range of 30 to 50 mm, and the height may be determined within the range of 10 to 50 mm.

Although the material for the applicator is not particularly limited, a material having strength enough to maintain the biasing force of the spring 40 is desirable. For example, the materials below can be used. As the materials for the housing 11 and cap 30, silicon, silicon dioxide, ceramic, a metal (e.g., stainless steel, titanium, nickel, molybdenum, chromium, or cobalt) may be used, in addition to a synthetic or natural resin material such as ABS resin, polystyrene, polypropylene, or polyacetal (POM). The piston 20 may be fabricated using the same material as that for microneedles.

Air vents for escaping air may be formed in the housing 11, cap 30, and piston 20. The configuration allows reduction in air resistance and weight.

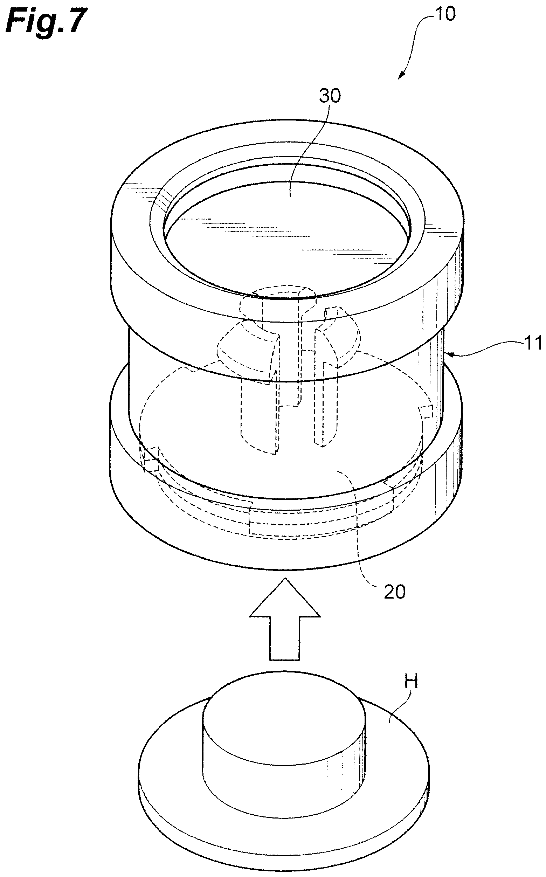

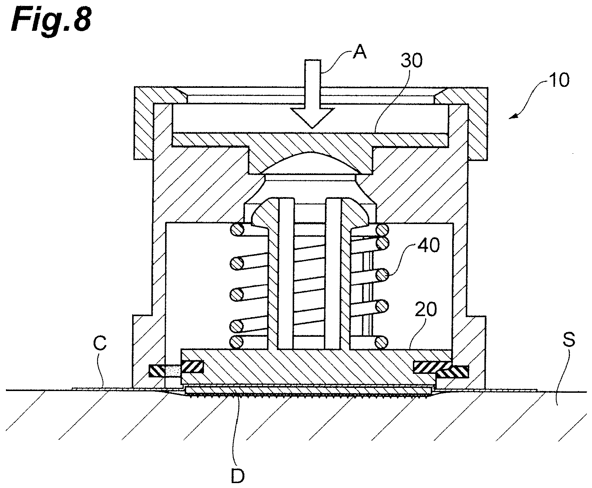

How to use the applicator 10 will be described with reference to Figures 7 and 8. Figure 7 is a view for explaining a case where an aid H is used during use of the applicator 10. Figure 8 is a view for explaining how to use the applicator 10.

First, the piston 20 is pushed toward the inside of the applicator 10 with a finger to fix the piston 20 against the biasing force of the spring 40. At this time, the piston 20 may be pushed up by hand or may be pushed up using the aid H, as shown in Figure 7. Note that the shape of the aid H is not limited to the example in Figure 7. With this operation, the applicator 10 enters the state shown in Figure 5(a). Since each claw portion 22a is formed so as to taper down toward an end of the piston rod 22, a user can engage the claw portions 22a with the claw receiving portion 16 with a small force to easily fix the piston 20 and can know completion of the fixation by a clicking noise. Force applied to fix the piston 20 can be increased or reduced by adjusting the slope of the taper shape on the lower side of the claw receiving portion 16.

As shown in Figure 8, the applicator 10 is positioned on microneedles D which is put on a skin S and held, and the cap 30 is pushed toward the inside of the applicator 10 (pushed in a direction of an arrow A) with a finger. Note that the microneedles D may be pasted to the skin with a covering agent C before the applicator 10 is positioned, as shown in Figure 8.

When the cap 30 is pushed, the projecting portion 31 provided at the lower surface pushes the claw portions 22a of the piston rods 22 toward the center of the through hole 13 to disengage the claw portions 22a from the claw receiving portion 16. As a result, the piston 20 is released from its fixed state, moves toward the outside of the applicator 10 by the biasing force of the spring 40, and collides with the microneedles D, as shown in Figure 8. The projecting portion 31 can thus be said to be a release mechanism for releasing the piston 20 fixed against the biasing force of the spring 40. Since the projecting portion 31 has the recessed portion 32 formed to have a mortar-shape, the claw portions 22a are drawn along the mortar-shape. The piston 20 (the applicator 10) is actuated only when a fixed force is applied to the cap 30. Accordingly, whoever performs administration, a fixed impact force is transmitted to the microneedles D, which allows reliable puncture (enhances the reproducibility of puncture).

Force required to release the piston 20 can be adjusted by changing the shape of the recessed portion 32 and that of the claw portions 22a. For example, if the slope of the taper shape of each claw portion 22a is made steeper, and the slope of the mortar-shape of the recessed portion 32 is adjusted accordingly, the piston 20 can be released from its fixed state with a small force.

With the above-described collision, the biasing force of the spring 40 is transmitted to the microneedles D via the piston 20, and microspikes of the microneedles D perforate the skin. An active ingredient applied to the microneedles D is administered into a body via the microspikes.

If the piston 20 is pushed toward the inside of the applicator 10 after the applicator 10 is used in the above-described manner, the piston 20 can be fixed again against the biasing force of the spring 40. The applicator 10 can thus be used any number of times.

As has been described above, according to the present embodiment, the cap 30 for releasing the piston 20 fixed against the biasing force of the spring 40 is provided to be freely movable in the extending direction of the housing 11. Accordingly, a member for fixing the cap 30 to the housing 11, a presser bar spring acting between an apparatus main body and the cap 30, and the like can be omitted, and the applicator can be reduced in size accordingly. The reduction in size of the applicator 10 allows reduction in the weight, which allows enhancement in portability.

Also, according to the present embodiment, the configuration in which the cap 30 is housed in the housing 11 reduces the dimension in the width direction (radial direction) of the applicator 10. This allows reduction in the size and weight of the applicator 10.

Additionally, if the claw receiving portion 16 and claw portions 22a constitute fixation means, and the projecting portion 31 with the recessed portion 32 constitutes a release mechanism, as in the present embodiment, it is possible to reduce the number of components required for an applicator and reduce the size and weight of the applicator accordingly.

(Second Embodiment)

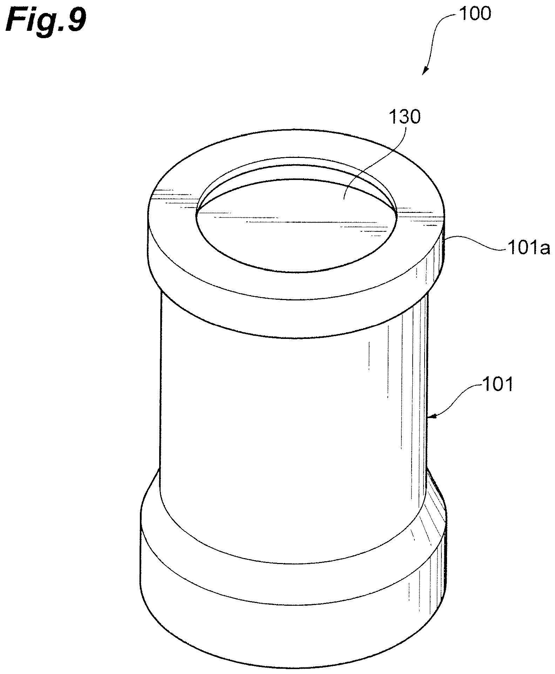

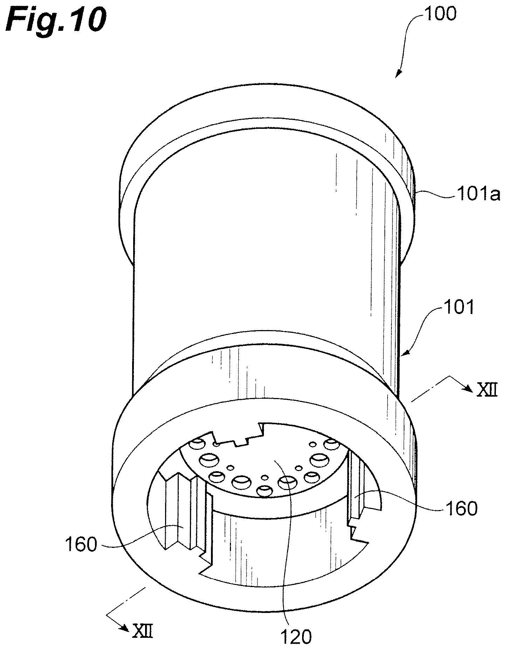

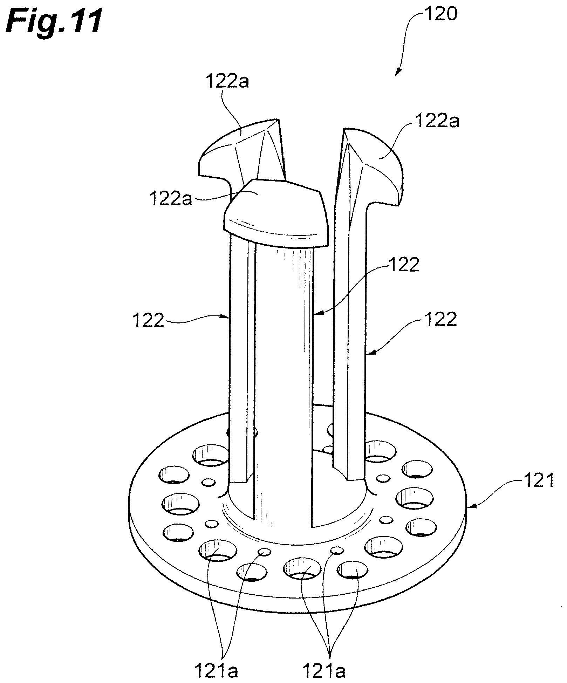

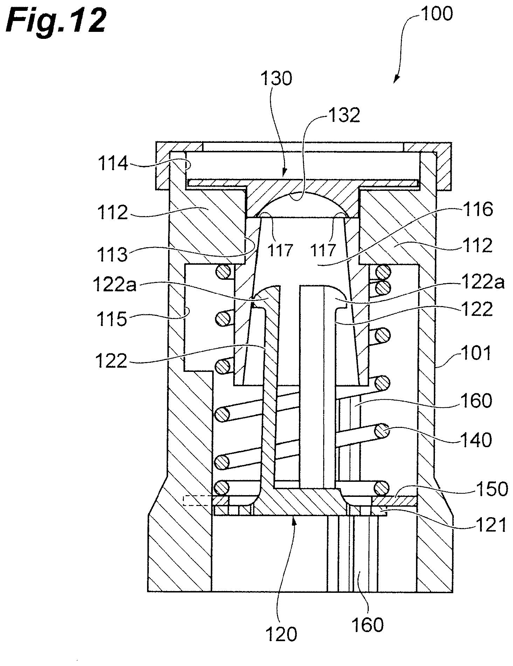

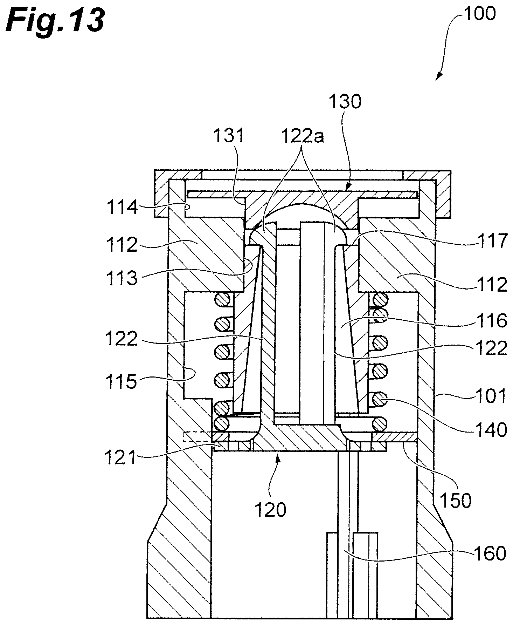

The structure of an applicator 100 according to a second embodiment will be described with reference to Figures 9 to 13. Figure 9 is a perspective view of the applicator 100 from above. Figure 10 is a perspective view of the applicator 100 from below. Figure 11 is a perspective view of a piston 120. Figure 12 is a cross-sectional view taken along line XII-XII in Figure 10. Figure 13 is a cross-sectional view showing a state in which claw portions shown in Figure 12 are fixed.

A housing 101 of the applicator 100 has a circular tube shape. In the applicator 100, the shape of the housing may be changed or a surface of the housing may be processed like the applicator 10, in view of ease of grip and ease of application of microneedles to a skin.

The piston 120 for impacting microneedles put on a skin is housed near one end (a second end) in the housing 101 (see Figure 10). A cap 130 for actuating the piston 120 is housed near the other end (a first end) in the housing 101 (see Figure 9). In the following description, the side where the cap 130 is housed is defined as the upper side of the applicator 100 or the housing 101, and the side where the piston 120 is housed is defined as the lower side of the applicator 100 or the housing 101. The housing 101 has an annular member 101a which fits thereon at the upper end and is tapered at the lower end to spread downward.

As shown in Figure 11, the piston 120 is composed of a disc-like piston plate 121 which is to collide with microneedles and a plurality of piston rods 122 which extend from near the center of an upper surface of the piston plate 121 in a direction orthogonal to the upper surface. A hooked claw portion 122a projecting outward in a radial direction of the piston plate 121 is formed at a distal end of each piston rod 122. The claw portion 122a tapers down toward the distal end of the piston rod 122. In the present embodiment, a plurality of air vents 121a for escaping air are formed in the piston plate 121.

The cap 130 is the same as the cap 30 according to the first embodiment and includes a projecting portion 131 having a recessed portion 132 shaped like a mortar.

A partition wall 112 for separating the inside into an upper portion and a lower portion is provided at a portion near the top of the inside of the housing 101. In the following description, a space on the upper side of the partition wall 112 is referred to as an upper space (a first space) 114 while a space on the lower side is referred to as a lower space (a second space) 115. A circular through hole 113 is formed in a central portion of the partition wall 112, and an inner tube 116 is attached to the through hole 113 while the inner tube 116 is inserted halfway from below the through hole 113 into the through hole 113. An inner wall of the inner tube 116 is tapered to spread downward and is responsible for guiding vertical movement of the piston rods 122.

An upper portion of the through hole 113 is responsible for guiding the projecting portion 131 of the cap 130 and has the same diameter as that of the projecting portion 131. An upper end of the inner tube 116 which is located near a central portion of the through hole 113 is responsible for engaging with the claw portions 122a of the piston rods 122 to fix the piston 120 and is hereinafter referred to as a claw receiving portion 117.

The piston 120, a compression spring (a biasing member or an elastic member, hereinafter to be simply referred to as a "spring") 140 which provides a biasing force to the piston 120, and an annular spring mount (support base) 150 which supports the spring 140 from below are housed in the lower space 115. A plurality of guides 160 for sliding the spring mount 150 in a vertical direction within a predetermined range are provided at an inner wall of the lower space 115.

Each guide 160 extends from the lower end of the housing 101 to near a lower end of the inner tube 116. A portion of the guide 160 in a lower approximately half region (a lower portion of the guide 160) is thicker than a portion in the remaining upper half region (an upper portion of the guide 160). The spring mount 150 has notches (not shown) to engage with the upper portions of the guides 160 formed in an outer periphery of the spring mount 150. The spring mount 150 is thus slidable in the vertical direction only on the upper portions of the guides 160. The spring 140 is housed in the lower space 115 so as to surround the inner tube 116. An upper end of the spring 140 is attached to a lower surface of the partition wall 112, and a lower end is in contact with an upper surface of the spring mount 150.

The piston 120 is housed in the lower space 115 after the spring 140 and spring mount 150 are housed in the lower space 115. The piston plate 121 has substantially the same diameter as an inner diameter which is limited by the lower portions of the guides 160 of the housing 101. Since the diameter of the piston plate 121 is larger than the inner diameter of the spring mount 150, the piston plate 121 is always located below the spring mount 150.

The piston 120 can be fixed against a biasing force of the spring 140 in the lower space 115. When the piston plate 121 is pushed from outside the housing 101 against the biasing force of the spring 140 in the applicator 100 in its initial state (see Figure 12), the claw portions 122a of the piston rods 122 pass through the inner tube 116 to be caught on the claw receiving portion 117, as shown in Figure 13. This causes the piston 120 to be fixed against the biasing force of the spring 140. Accordingly, the claw receiving portion 117 and the claw portions 122a of the piston rods 122 can be said to be fixation means.

The cap 130 is housed in the upper space 114 such that the projecting portion 131 faces the partition wall 112. After the whole of the cap 130 is housed in this manner, the annular member 11a is attached to an end of the cap 130. The configuration prevents the cap 130 from popping out of the housing 101. A method for housing the cap 130 is the same as in the first embodiment. Accordingly, the cap 130 is provided to be freely movable in an extending direction of the housing 101 (the vertical direction) in the upper space 114 such that the projecting portion 131 can contact the claw portions 122a when the piston 120 is fixed by claw portions 122a.

Parameters related to the spring 140 and piston 120 can be defined in the same manner as in the first embodiment. In the present embodiment as well, the spring- and piston-related theoretical formulae (1) to (4) described above hold.

The dimensions of the applicator 100 may be determined according to the dimensions of microneedles, like the first embodiment, or may be determined from another point of view. The material for the applicator 100 can be selected in the same manner as in the first embodiment.

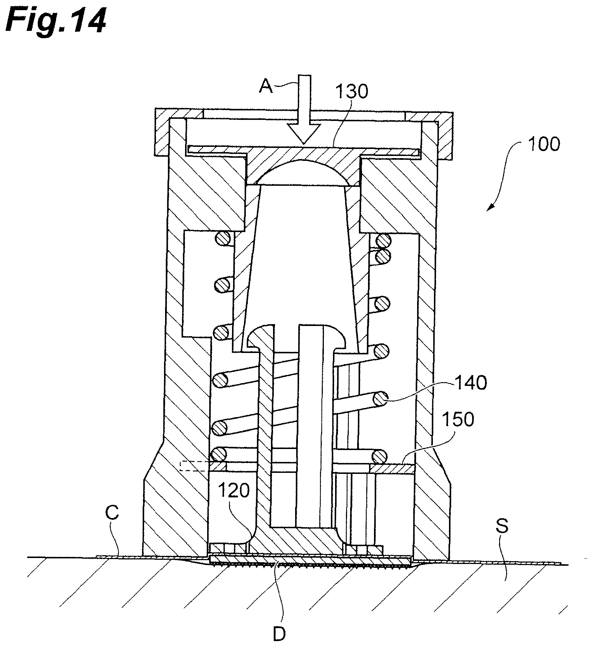

How to use the applicator 100 will be described with reference to Figures 12 to 14. Figure 14 is a view for explaining how to use the applicator 100.

The initial state of the applicator 100 is as shown in Figure 12. First, the piston 120 is pushed toward the inside of the applicator 100 with a finger to fix the piston 120 against the biasing force of the spring 140. Note that the piston 120 may be pushed up by hand or may be pushed up using an aid, like the first embodiment. The applicator 100 with the fixed piston 120 is in the state shown in Figure 13. Since the inner wall of the inner tube 116 is formed so as to taper down toward the upper end (the claw receiving portion 117), a user can engage the claw portions 122a with the claw receiving portion 117 with a small force to easily fix the piston 120. The user can also know completion of the fixation by a clicking noise. Force applied to fix the piston 120 can be increased or reduced by adjusting the slope of the taper shape of the inner tube 116.

As shown in Figure 14, the applicator 100 is positioned on microneedles D which is put on a skin S and held, and the cap 130 is pushed toward the inside of the applicator 100 (pushed in a direction of an arrow A) with a finger. Note that the microneedles D may be pasted to the skin with a covering agent C in the present embodiment as well.

When the cap 130 is pushed, the projecting portion 131 provided at a lower surface pushes the claw portions 122a of the piston rods 122 toward the center of the through hole 113 to disengage the claw portions 122a from the claw receiving portion 117. As a result, the piston 120 is released from its fixed state and moves toward the outside of the applicator 100 by the biasing force of the spring 140. The projecting portion 131 can thus be said to be a release mechanism for releasing the piston 120 fixed against the biasing force of the spring 140.

The arrangement in which the piston 120 is actuated by the cap 130 is the same as in the first embodiment. In the present embodiment as well, any user can apply a fixed impact force to the microneedles D to enhance the reproducibility of puncture. Note that since the spring mount 150 slides only on the upper portions of the guides 160 in the present embodiment, the piston 120 moves under the biasing force of the spring 140 via the spring mount 150 only in a zone along the upper portions of the guides 160 (a second zone) (the piston 120 is pushed by the spring mount 150 in the second zone). After the spring mount 150 stops at a lower end of the second zone, the piston 120 moves in a zone along the lower portions of the guides 160 (a first zone) without being subjected to the biasing force of the spring 140. The piston 120 collides with the microneedles D at a lower end of the applicator 100 (see Figure 14).

Like the first embodiment, force required to release the piston 120 can be adjusted by changing the shape of the recessed portion 132 and that of the claw portions 122a. For example, if the slope of the taper shape of each claw portion 122a is made steeper, and the slope of the mortar-shape of the recessed portion 132 is adjusted accordingly, the piston 120 can be released from its fixed state with a small force.

With the above-described collision, the biasing force of the spring 140 is transmitted to the microneedles D via the piston 120, and microspikes of the microneedles D perforate the skin. An active ingredient applied to the microneedles D is administered into a body via the microspikes.

If the piston 120 is pushed toward the inside of the applicator 100 after the applicator 100 is used in the above-described manner, the piston 120 can be fixed again against the biasing force of the spring 140. The applicator 100 can thus be used any number of times.

The present embodiment can obtain the same effects as those of the first embodiment. According to the present embodiment, the cap 130 for releasing the piston 120 fixed against the biasing force of the spring 140 is provided to be freely movable in the extending direction of the housing 101. Accordingly, a member for fixing the cap 130 to the housing 101, a presser bar spring acting between an apparatus main body and the cap 130, and the like can be omitted, and the applicator can be reduced in size accordingly. The reduction in size of the applicator 100 allows reduction in the weight, which allows enhancement in portability.

Also, according to the present embodiment, the configuration in which the cap 130 is housed in the housing 101 reduces the dimension in a width direction (radial direction) of the applicator 100. This allows reduction in the size and weight of the applicator 100.

Additionally, if the claw receiving portion 117 and claw portions 122a constitute fixation means, and the projecting portion 131 with the recessed portion 132 constitutes a release mechanism, as in the present embodiment, it is possible to reduce the number of components required for an applicator and reduce the size and weight of the applicator accordingly.

**Example**

The present invention will be concretely described below with reference to an example. The present invention, however, is not limited in any way to the example.

The puncturability of two applicators corresponding to the above-described applicators 10 and 100 were compared. The puncturability of each applicator was evaluated by administering ovalbumin (OVA) in vitro (into a human skin) using microneedles and obtaining the amount of OVA transferred to the human skin. An amount transferred here refers to the amount of part administered into the human skin of OVA stuck to the microneedles. In the following description, the applicator having the same configuration as that of the applicator 10 (the first embodiment) is referred to as "type A" while the applicator having the same configuration as that of the applicator 100 (the second embodiment) is referred to as "type B." For types A and B, a common spring having a spring constant k of 6374.5 N/m was used.

The microneedles was made of polylactic acid, the area of a substrate was 1.13 cm², and the number of microspikes (needles) was 640. The height of each microspike was 500 $\mu$m, and the area of a flat portion at a distal end of the microspike was 64 to 144 $\mu$m². A range within which each microspike is coated when OVA is applied to the microspike was about 180 $\mu$m including the top of the microspike. The total amount (initial content) of OVA applied to the microneedles was 65.2 ± 6.3 $\mu$g.

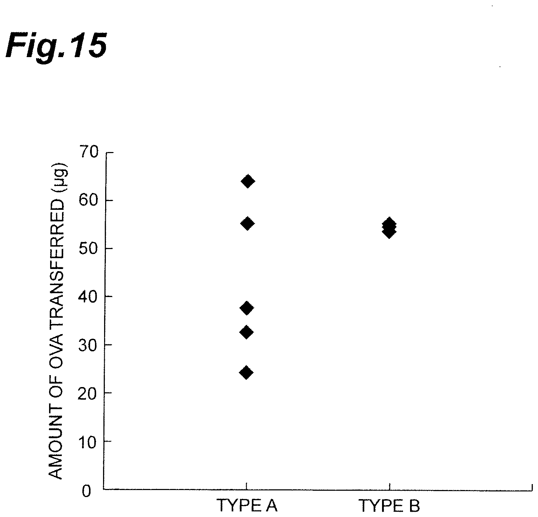

OVA was extracted by immersing the microneedles peeled off from the human skin after administration of OVA in a phosphate buffered saline solution (PBS), and an amount transferred was obtained by subtracting the amount of OVA extracted from the initial amount applied. This experiment was performed a plurality of times for each of types A and B, and the results shown in Figure 15 were obtained. Figure 15 is a graph showing amounts transferred for each of types A and B.

As shown in Figure 15, it was found that the amounts transferred for type B were more uniform than the amounts transferred for type A and that the reproducibility of puncture was higher. In type A, since a lower end of a spring is in contact with an upper surface of a piston plate, when a piston collides with microneedles, the spring pushes the piston against a skin, which results in a resilient force acting on an applicator main body. In contrast, in type B, since a lower end of a spring is in contact not with a piston plate but with a spring mount, the spring stops extending halfway along the applicator, and only the piston moves to a lower end of the applicator after the stop to collide with the microneedles. At the time of the collision, the piston is separate from an applicator main body and is not subjected to a reaction at the time of the collision. Accordingly, use of type B in which a reaction on the spring is blocked from being transmitted to the application main body allows further enhancement of the reproducibility of puncture.

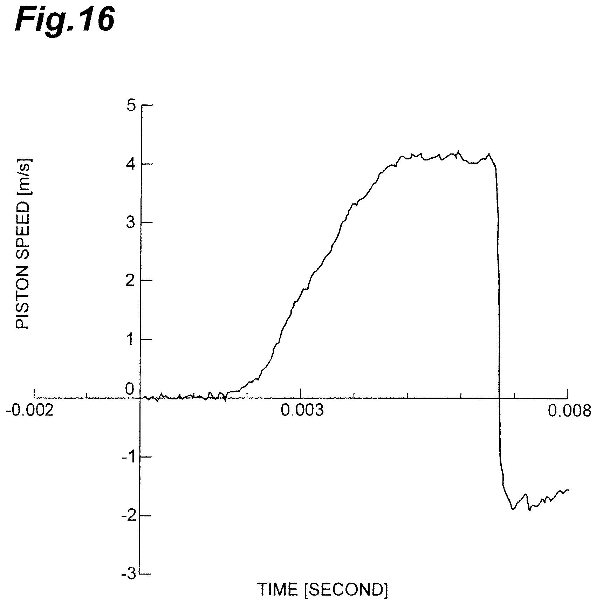

Note that a velocity profile of the piston in type B is shown in the graph in Figure 16. The ordinate and abscissa of the graph represent piston speed (m/s) and time (sec) elapsed since the start of the push of a cap, respectively. The speed remains constant at about 4 m/s from about 0.05 sec to before 0.07 sec. This indicates that the piston moved without being subjected to a biasing force of the spring during the time period.

The present invention has been described above in detail with reference to specific embodiments. The present invention, however, is not limited to the above-described embodiments. Various modifications can be made to the present invention without departing from the scope thereof.

Although the above-described embodiments use a spring as a biasing member, a biasing member is not limited to this. For example, a mechanism which causes a piston to collide with microneedles by a jet of compressed gas may be adopted as a biasing member.

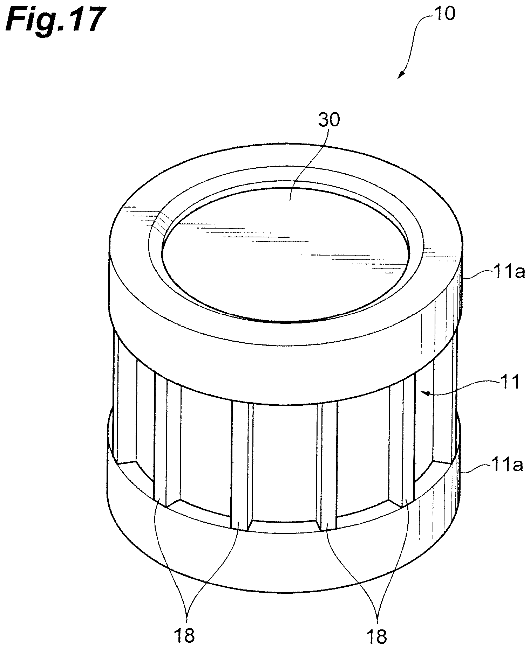

In the above-described first embodiment, the housing 11 is shaped such that the outer wall is smooth. As shown in Figure 17, rod-like raised portions 18 formed to extend along the extending direction of the housing 11 may be one-dimensionally arranged at intervals on the outer wall of the housing 11. Use of the housing 11 allows reduction in collision noise generated when the actuated piston 20 stops at the end of the housing 11. Note that such raised portions can also be provided in the applicator 100 according to the second embodiment in the same manner.

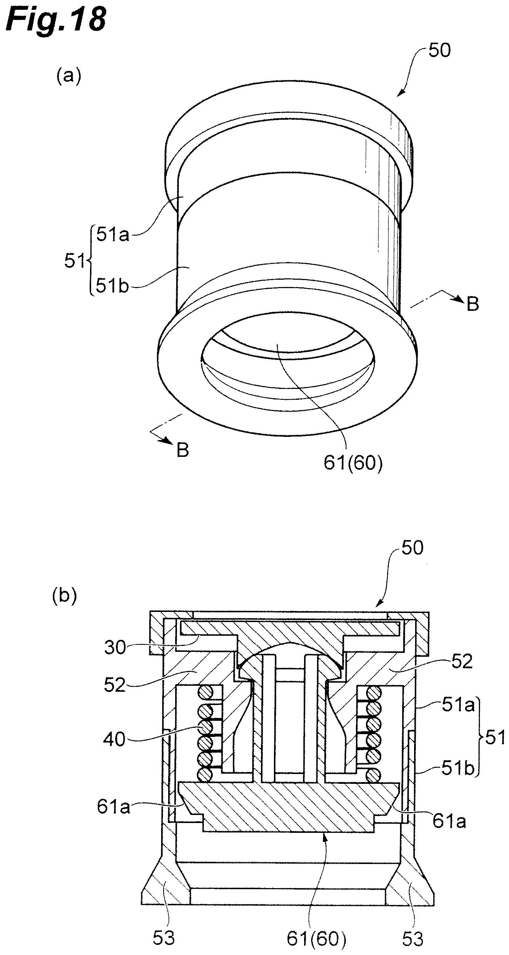

The configuration of an applicator may be one shown in Figures 18. A housing 51 of an applicator 50 shown in Figures 18 is composed of an upper portion 51a having a partition wall 52 which is the same as the partition wall 12 according to the first embodiment and a lower portion 51b. The applicator 50 is completed by housing a cap 30 in the upper portion 51a of the housing 51 and a piston 60 and a spring 40 in the lower portion 51b of the housing 51 and coupling the lower portion 51b and upper portion 51a. A rim 53 forming a lower opening of the housing 51 is tapered both on the outer side and on the inner side in a radial direction to spread downward. The piston 60 and the piston 20 according to the first embodiment are the same in the configuration of piston rods but are different in the configuration of a piston plate. A rim 61a of a piston plate 61 is formed so as to contact the inner side of the rim 53 during actuation of the piston 60, which prevents the piston 60 from popping out of the housing 51. Such a modification can also be applied to the applicator 100 according to the second embodiment.

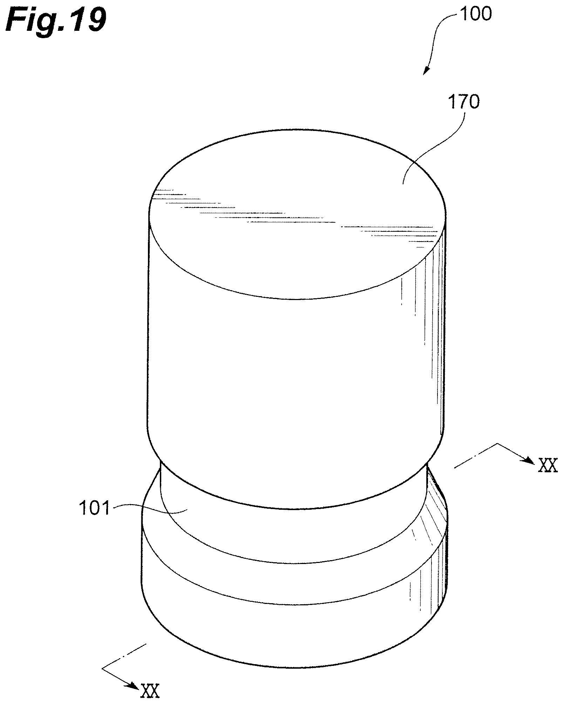

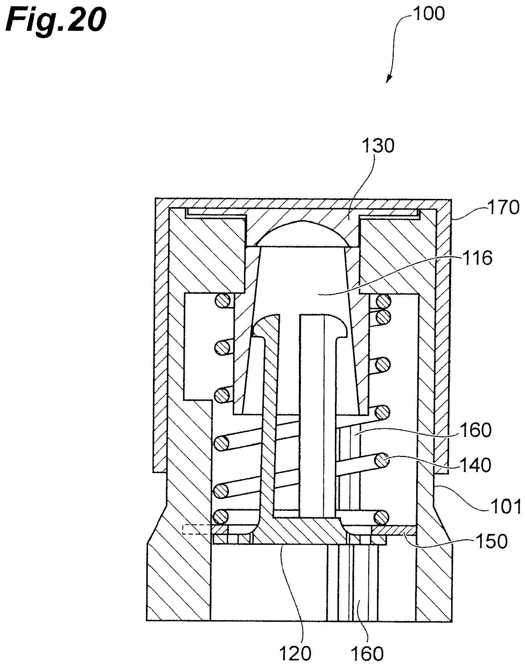

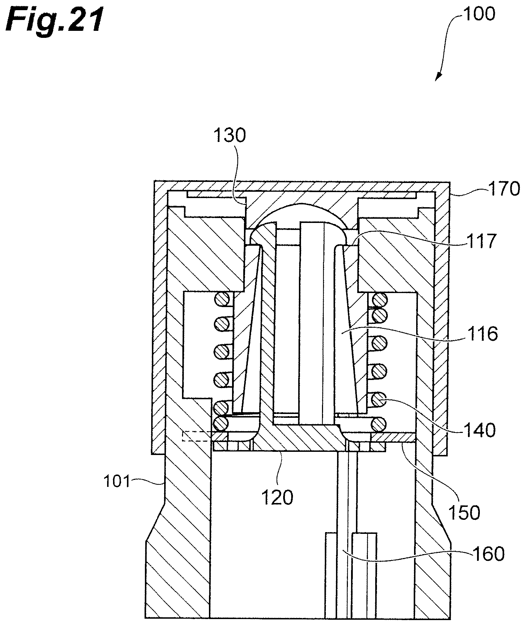

The applicator 100 according to the second embodiment may be modified in a manner as shown in Figures 19 to 21. Note that Figures 19 to 21 correspond to Figures 9, 12, and 13. The applicator 100 according to this modification has not the annular member 101a but a hood 170 which covers an upper portion of the housing 101. As shown in Figures 20 and 21, the cap 130 is attached to an upper wall of the hood 170, and the hood 170 and cap 130 are integrated together. Note that the hood 170 together with the cap 130 may be referred to as a "cap."

Introduction of the hood 170 allows a user to push the cap 130 down by gripping the hood 170 and moving the hood 170 downward. Accordingly, the piston 120 can be actuated more easily (e.g., easily with one hand). Additionally, since the piston 120 can be actuated by pushing the cap 130 with a favorable load (e.g., 400 to 600 g or about 500 g), a fixed biasing force can be transmitted to microneedles without applying an excess load to a skin on which the applicator 100 is put. Such a hood can also be applied to the applicator 10 according to the first embodiment.

## Reference Signs List

| 10, 50, 100 | applicator |
|---|---|
| 11, 51, 101 | housing |
| 12, 52, 112 | partition wall |
| 13, 113 | through hole |
| 14, 114 | upper space |
| 15, 115 | lower space |
| 16, 117 | claw receiving portion |
| 17 | cushioning material |
| 18 | raised portion |
| 20, 60, 120 | piston |
| 21, 61, 121 | piston plate |
| 21a | projecting portion |
| 22, 122 | piston rod |
| 22a, 122a | claw portion |
| 23 | cushioning material |
| 30, 130 | cap |
| 31, 131 | projecting portion |
| 32, 132 | recessed portion |
| 40, 40A, 140 | spring |
| 116 | inner tube |
| 121a | air vent |
| 150 | spring mount |
| 160 | guide |
| 170 | hood |

## Claims

1. An applicator (10) for applying a microneedle to a skin, comprising:

   a tubular housing (11);
   a piston (20) housed in a lower space (15) of the housing (11);
   fixation means (16, 22a) for fixing the piston (20) against a biasing force of a biasing member (40) in the housing (11); and
   a cap (30) provided near a first end of the housing (11), the cap (30) having a release mechanism (31) for releasing the piston (20) fixed by the fixation means (16, 22a) at a surface facing the piston (20), wherein
   the cap (30), which is not fixed in an upper space (14) of the housing (11), is provided to be freely movable in a vertical direction of the housing (11) in the upper space (14) such that the release mechanism (31) can contact the fixation means (16, 22a) when the piston (20) is fixed by the fixation means (16, 22a); and
   the biasing member (40) is an elastic member, **characterized in that** the piston (20) moves without being subjected to the biasing force of the elastic member (40) in a first zone which is a part of a movement zone for the piston (20) moving toward the skin.

2. The applicator (10) according to claim 1, wherein a whole of the cap (30) is housed in the housing (11).

3. The applicator (10) according to claim 1 or 2, wherein the biasing member (40) is a conical spring (40A).

4. The applicator (10) according to any one of claims 1 to 3, wherein a partition wall (12) for separating the upper space (14) housing the release mechanism (31) of the cap (30) and the lower space (15) housing the piston (20) from each other is provided in the housing (11), and the fixation means (16, 22a) is composed of a rim of a through hole (13) which is formed in the partition wall (12) and a claw portion (22a) which is provided at an end of a piston rod (22) of the piston (20) and can engage with the rim.

5. The applicator (10) according to claim 4, wherein the claw portion (22a) is formed to taper down toward the end of the piston rod (22).

6. The applicator (10) according to claim 4 or 5, wherein the release mechanism (31) is a projecting portion (31) which is provided at the surface of the cap (30) facing the piston (20), and a recessed portion (32) for disengaging the claw portion (22a) from the rim of the partition wall (12) by pushing the claw portion (22a) toward a center of the through hole (13) is formed in the projecting portion (31).

7. The applicator (10) according to claim 6, wherein the recessed portion (32) formed in the projecting portion (31) has a mortar-shape.

8. The applicator (100) according to any one of claims 1 to 7, further comprising a support base (150) which supports the elastic member (140) and transmits the biasing force to the piston (120) in a second zone different from the first zone, wherein
the support base (150) that pushes the piston (120) by the biasing force stops at one end of the second zone to allow the piston (120) to move without being subjected to the biasing force in the first zone.

**Patentansprüche**

1. Ein Applikator (10) zum Aufbringen einer Mikronadel auf eine Haut, umfassend:

ein rohrförmiges Gehäuse (11);
einen Kolben (20), der in einem unteren Raum (15) des Gehäuses (11) untergebracht ist;
Fixierungsmittel (16, 22a) zum Fixieren des Kolbens (20) gegen eine Vorspannkraft eines Vorspannelementes (40) im Gehäuse (11); und
eine Kappe (30), die in der Nähe eines ersten Endes des Gehäuses (11) vorgesehen ist,
wobei die Kappe (30) einen Freigabemechanismus (31) zum Freigeben des durch die Fixierungsmittel (16, 22a) fixierten Kolbens (20) an einer dem Kolben (20) zugewandten Fläche aufweist,
wobei die Kappe (30), die nicht in einem oberen Raum (14) des Gehäuses (11) befestigt ist, vorgesehen ist, um in einer vertikalen Richtung des Gehäuses (11) in dem oberen Raum (14) frei beweglich zu sein, so dass der Freigabemechanismus (31) das Fixierungsmittel (16, 22a) kontaktieren kann, wenn der Kolben (20) durch das Fixierungsmittel (16, 22a) fixiert ist; und das Vorspannelement (40) ein elastisches Element ist, **dadurch gekennzeichnet, dass**
der Kolben (20) sich bewegt, ohne der Vorspannkraft des elastischen Elements (40) ausgesetzt zu sein, in einer ersten Zone, die ein Teil einer Bewegungszone für den Kolben (20) ist, der sich in Richtung der Haut bewegt.

2. Der Applikator (10) nach Anspruch 1, wobei die Kappe (30) vollständig in dem Gehäuse (11) untergebracht ist.

3. Der Applikator (10) nach Anspruch 1 oder 2, wobei das Vorspannelement (40) eine konische Feder (40A) ist.

4. Der Applikator (10) nach einem der Ansprüche 1 bis 3, wobei im Gehäuse (11) eine Trennwand (12) vorgesehen ist, die den oberen, den Freigabemechanismus (31) der Kappe (30) aufnehmenden Raum (14) und den unteren, den Kolben (20) aufnehmenden Raum (15) voneinander trennt, und das Fixierungsmittel (16, 22a) aus einem Rand eines Durchgangslochs (13), das in der Trennwand (12) ausgebildet ist, und einem Klauenabschnitt (22a) gebildet ist, der an einem Ende einer Kolbenstange (22) des Kolbens (20) vorgesehen ist und in den Rand eingreifen kann.

5. Der Applikator (10) nach Anspruch 4, wobei der Klauenabschnitt (22a) so ausgebildet ist, dass er sich zum Ende der Kolbenstange (22) hin verjüngt.

6. Der Applikator (10) nach Anspruch 4 oder 5, wobei der Freigabemechanismus (31) ein vorstehender Abschnitt (31) ist, der an der dem Kolben (20) zugewandten Oberfläche der Kappe (30) vorgesehen ist, und
ein vertiefter Abschnitt (32) zum Lösen des Klauenabschnitts (22a) von dem Rand der Trennwand (12) durch Drücken des Klauenabschnitts (22a) in Richtung eines Zentrums des Durchgangslochs (13) in dem vorstehenden Abschnitt (31) ausgebildet ist.

7. Der Applikator (10) nach Anspruch 6, wobei der in dem vorstehenden Abschnitt (31) ausgebildete vertiefte Abschnitt (32) eine Mörserform aufweist.

8. Applikator (100) nach einem der Ansprüche 1 bis 7, ferner umfassend eine Stützbasis (150), die das elastische Element (140) stützt und die Vorspannkraft auf den Kolben (120) in einer zweiten, von der ersten Zone verschiedenen Zone überträgt, wobei die Stützbasis (150), die den Kolben (120) durch die Vorspannkraft drückt, an einem Ende der zweiten Zone anhält, damit sich der Kolben (120) bewegen kann, ohne der Vorspannkraft in der ersten Zone ausgesetzt zu sein.

**Revendications**

1. Applicateur (10) d'application d'une micro-aiguille à une peau, comprenant :

un logement (11) tubulaire ;
un piston (20) logé dans un espace inférieur (15) du logement (11) ;
un moyen de fixation (16, 22a) pour fixer le piston (20) contre une force de sollicitation d'un élément de sollicitation (40) dans le logement (11) ; et

un capuchon (30) disposé près d'une première extrémité du logement (11), le capuchon (30) ayant un mécanisme de libération (31) pour libérer le piston (20) fixé par le moyen de fixation (16, 22a) au niveau d'une surface faisant face au piston (20),

le capuchon (30), qui n'est pas fixé dans un espace supérieur (14) du logement (11), étant disposé pour être librement mobile dans un sens vertical du logement (11) dans l'espace supérieur (14) de sorte que le mécanisme de libération (31) peut entrer en contact avec le moyen de fixation (16, 22a) lorsque le piston (20) est fixé par le moyen de fixation (16, 22a) ; et

l'élément de sollicitation (40) étant un élément élastique, **caractérisé en ce que** le piston (20) se déplace sans être soumis à la force de sollicitation de l'élément élastique (40) dans une première zone qui est une partie d'une zone de mouvement pour le piston (20) se déplaçant vers la peau.

2. Applicateur (10) selon la revendication 1, l'entièreté du capuchon (30) étant logée dans le logement (11).

3. Applicateur (10) selon la revendication 1 ou 2, l'élément de sollicitation (40) étant un ressort conique (40A).

4. Applicateur (10) selon l'une quelconque des revendications 1 à 3,
une paroi de partition (12) pour séparer, l'espace supérieur (14) logeant le mécanisme de libération (31) du capuchon (30) et l'espace inférieur (15) logeant le piston (20), l'un de l'autre étant prévue dans le logement (11), et
le moyen de fixation (16, 22a) étant composé d'un rebord d'un trou traversant (13) qui est formé dans la paroi de partition (12) et d'une portion griffe (22a) qui est disposée à une extrémité d'une tige de piston (22) du piston (20) et qui peut venir en prise avec le rebord.

5. Applicateur (10) selon la revendication 4,
la portion griffe (22a) étant formée pour s'effiler de manière descendante vers l'extrémité de la tige de piston (22).

6. Applicateur (10) selon la revendication 4 ou 5, le mécanisme de libération (31) étant une portion de projection (31) qui est disposée à la surface du capuchon (30) faisant face au piston (20), et
une portion renfoncée (32) pour le désengagement de la portion griffe (22a) du rebord de la paroi de partition (12) en poussant la portion griffe (22a) vers un centre du trou traversant (13) étant formée dans la portion de projection (31).

7. Applicateur (10) selon la revendication 6,
la portion renfoncée (32) formée dans la portion de projection (31) ayant une forme de mortier.

8. Applicateur (100) selon l'une quelconque des revendications 1 à 7, comprenant en outre une base de support (150) qui supporte l'élément élastique (140) et qui transmet la force de sollicitation au piston (120) dans une seconde zone différente de la première zone,
la base de support (150) qui pousse le piston (120) par la force de sollicitation s'arrêtant à une extrémité de la seconde zone pour permettre au piston (120) de se déplacer sans être soumis à la force de sollicitation dans la première zone.

*Fig.1*

*Fig.2*

# *Fig.3*

*Fig.4*

# Fig.5

(a)

(b)

*Fig.6*

(a)

(b)

**_Fig.7_**

**Fig.8**

# Fig.9

100

130

101a

101

**Fig.10**

*Fig.11*

# Fig.12

*Fig.13*

## Fig.14

*Fig.15*

## *Fig.16*

## Fig.17

# Fig.18

(a)

(b)

*Fig.19*

**Fig.20**

## Fig.21

**EP 2 626 107 B1**

## REFERENCES CITED IN THE DESCRIPTION

**Patent documents cited in the description**

- JP 4198985 B
- JP 2007509706 A
- JP 2010501211 A
- JP 2010233803 A
- US 20050261631 A1
- JP 2005533625 A